# EUROPEAN PATENT APPLICATION

(11) **EP 4 599 840 A1**
(43) Date of publication of application: **13.08.2025**
(21) Application number: 24156650.4
(22) Date of filing: 08.02.2024
(51) Int. Cl.: A61K 38/16, A61P 31/02, A61P 31/04, A61P 31/10, A61P 33/00, C07K 14/415, C12N 1/20, C12N 15/82

(54) **ANTIMICROBIAL PROTEINS**

(71) Applicant: THE OLIVE FEED CORPORATION LIMITED, A96 D8C2 Co. Dublin Glenageary (IE)
(72) Inventor: DUNNE, Gavin, Loughlinstown, Genageary Co. Dublin A96 D8C2 (IE); KNOX, Andrew, Loughlinstown, Genageary Co. Dublin A96 D8C2 (IE)
(74) Representative: Tomkins & Co

(57) **Abstract**

Described are antimicrobial proteins, a nucleic acid sequence encoding the said protein, a gene encoding the said protein or comprising the said nucleic acid sequence, a host cell comprising the said nucleic acid sequence or the said gene, a composition comprising the antimicrobial protein and the use of the antimicrobial protein or the composition as antiseptic agent.

## Description

### Field of the Invention

The present invention relates to antimicrobial proteins, to a nucleic acid sequence encoding the said protein, a gene encoding the said protein or comprising the said nucleic acid sequence, to a host cell comprising the said nucleic acid sequence or the said gene, to a composition comprising the antimicrobial protein and to the use of the antimicrobial protein or the composition as antiseptic agent.

### Background to the Invention

Antimicrobial proteins are known, e.g. from International Patent publication No. WO 00/71735. However, the increasing global population and mobility, along with climate change, are contributing to the spread of infectious diseases. New antimicrobials are needed to address emerging pathogens. Further, microbes tend to develop resistance against antimicrobial agents and traditional antibiotics are often ineffective, for example against biofilms and chronic infections. New antimicrobials, especially those targeting biofilms and so-called persisters, are required to treat these chronic infections. Therefore, there is an ongoing demand for the provision of new antimicrobial agents such as new proteins having antimicrobial activity.

### Summary of the Invention

The present invention provides new antimicrobial proteins according to the main claim, i.e., comprising the amino acid sequence of SEQ ID No. 1 or No. 2, or a protein being a functional analogue of these proteins comprising an amino acid sequence having at least 90% amino acid sequence homology with these amino acid sequences.

Herein, the term `antimicrobial' refers to the ability to inhibit the growth or kill microorganisms, including bacteria (antibacterial), viruses (antiviral) , fungi (antifungal), and/or parasites (antiparasitic). The antimicrobial protein has at least one of these antibacterial, antiviral, antifungal, and antiparasitic activities, preferably at least two, more preferably at least three, and most preferably all four of these activities.

The term 'functional analogue' is intended to mean a protein having at least 90% amino acid sequence homology with the protein comprising the amino acid sequence of SEQ ID No. 1 or No. 2, having the same or similar antimicrobial activity.

Proteins of SEQ ID Nos. 1 and 2 are herein referred to as 'defensin 1' or `OIv1' and defensin 2' or 'Olv2', respectively. In view of the relative small size of the proteins (e.g., 50 amino acids for Olv2) the term 'peptide' would be suitable as well, and could also be used to refer to the proteins of the invention.

Sequence homology refers to the similarity in the primary structure of biological molecules, such as amino acid sequences in proteins. In the context of amino acids, sequence homology indicates that two or more protein sequences share a significant degree of similarity or identity in the arrangement of their constituent amino acids, including deletion, insertion or substitution of one or more amino acids. Sequence homology can be quantified using various bioinformatics tools and algorithms that align the amino acid sequences of proteins to identify matching or similar regions. The results of these analyses are expressed as a percentage of identity or similarity, indicating the proportion of amino acids that are the same or similar between the compared sequences. The functional analogy preferably comprises an amino acid sequence having an amino acid sequence homology with that of SEQ ID No 1 or SEQ ID No 2 of at least 91%, 92%, 93%, 94%, 95%, 96%, 97%, 98% or 99%. The protein preferably comprises the amino acid sequence of SEQ ID No. 1 or SEQ ID No. 2.

The protein preferably has a length of not more than 100 amino acids, preferably not more than 90, 80, 70, 60 amino acids, and most preferably consists of the amino acid sequence of SEQ ID No. 1 or SEQ ID No. 2.

The new antimicrobial proteins have been identified by using a suite of bioinformatic tools: BLASTp for protein sequence alignment, Levenshtein distance for sequence similarity assessment, and HMMER3 for profile-based hidden Markov model searches, and by subsequent analysis of the transcriptomic data of *Olea europaea.* The Antimicrobial Peptide Database (APD3) served as reference dataset. By cloning the cDNA encoding the proteins of the invention from RNA of *Olea europaea* and isolation of the proteins, the antimicrobial activity was found using a *Candida* growth inhibition assay, as will be explained in more detail in the examples. Said growth inhibition assay can also be used to identify functional analogues of the invention, wherein the activity of the analogue should be not less than 80% of the antimicrobial activity of the corresponding protein comprising the amino acid sequence of SEQ ID No. 1 or SEQ ID No. 2.The said activity is preferably not less than 85%, more preferably not less than 90% and even more preferable not less than 95%, and most preferably equals that of the corresponding protein comprising the amino acid sequence of SEQ ID No. 1 or SEQ ID 2.

Since the proteins of the invention were identified from transcriptomic data from *Olea europaea,* the antimicrobial protein is preferably derived from *Olea europaea.* However, other plants may have similar antimicrobial proteins or functional analogues.

The invention also relates to a nucleic acid sequence, encoding the protein of the invention. The skilled person, knowing the amino acid sequence of the protein as defined above is well aware of deducting a suitable nucleic acid, encoding the said protein, based on the genetic code, its degeneracy and the codon preference of any organism envisaged for expression of the encoding nucleic acid sequence.

Accordingly, the invention also encompasses a gene, encoding the protein of the invention or comprising the above-described nucleic acid sequence.

The invention also relates to a nucleic acid vector, comprising the above nucleic acid sequence or gene. The vector is preferably a DNA plasmid, comprising gene expression elements suitable to be used by an envisaged host. The gene or coding sequence can be incorporated in the plasmid preceded by a leader sequence, or by a gene insert encoding a fusion protein partner, resulting in expression of the antimicrobial protein as a fusion protein.

The invention further encompasses a host cell, comprising the coding nucleic acid sequence or gene as described above, wherein the said nucleic acid sequence or gene is exogenous to the host cell, i.e. originating from another organism. The host cell is preferably transformed with the above-described nucleic acid vector. The skilled person is aware of suitable host cells, such as yeast cells and bacterial cells. In an attractive embodiment, the host cell is a bacterium, and preferably belongs to the genus *Escherichia,* more particular *Escherichia coli.*

The invention also relates to a composition, comprising the antimicrobial protein of the invention. The skilled person is aware of formulating a suitable composition, using one or more additional components such as a carrier, preservatives and the like and any combination thereof. Such a composition may be in pharmaceutically acceptable form. Any additional component of the compositions may be chosen to be pharmaceutically acceptable. The composition may be a liquid or solid. The composition is preferably an aqueous composition, i.e., based on water as solvent. Such a composition may comprise one or more cosolvents, such as glycerine or ethanol or the like. Such compositions can e.g. be used for cleaning any surface such as those of working surfaces, medical instruments and the like. The composition may comprise one or more additional antimicrobial agents to provide a cocktail of active substances. Also compositions can be formulated as a medicament against a microbial infection, e.g. for topical administration in the form of a cream, ointment or lozenge or any other form, suitable for an envisaged administration. For example, an oral administration form can be in the form of a liquid formulation, or in the form of a tablet etc.

The protein can be produced by the host as described above and subsequently purified to the desired extent, or can be made by known *in vitro* protein synthesis techniques.

The invention also relates to the use of the antimicrobial protein, or the composition as antiseptic agent.

In a desired embodiment, the antimicrobial protein is provided for use for treatment of a microbial infection in a patient in need thereof. The infection can originate from any known bacterium, virus, fungus or parasite, against which the antimicrobial agent shows the antimicrobial effect.

### Brief Description of the Drawings

Embodiments of the invention will be described, by way of example only, with reference to the accompanying drawings in which:

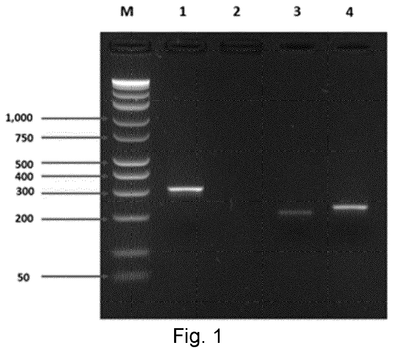

**Figure 1** shows a gel electrophoresis on PCR products for genes of interest in an *Olea europaea* cDNA library. Lane (M) molecular marker, lane 1: Ribosomal 18s subunit RNA Control (positive control) Lane 2: No reverse transcriptase control for 18s RNA (negative control). Lane 3 *O*. *europaea* defensin 1 (Olv1); Lane 4, *O*. *europaea* defensin 2 (Olv2).

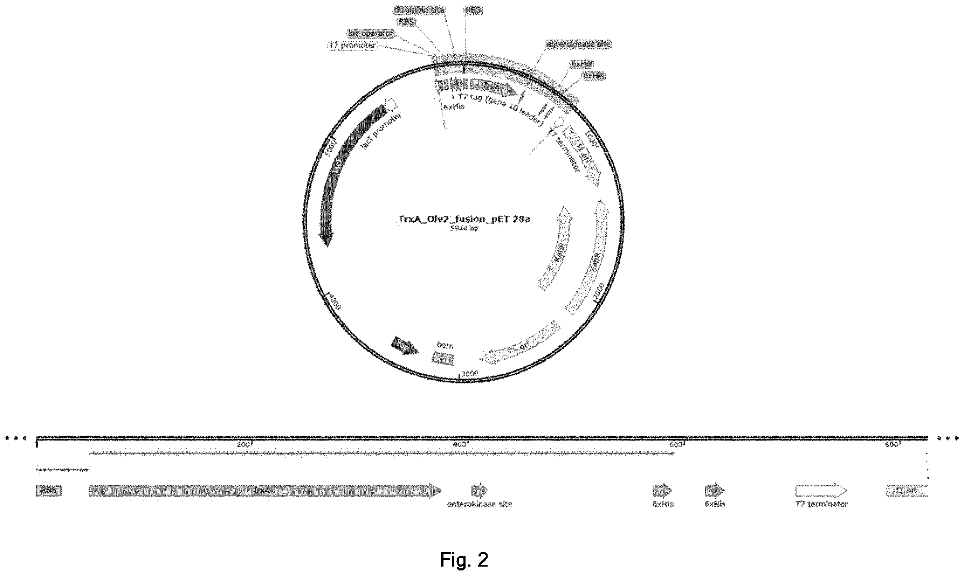

**Figure 2** shows the pET28a (+) vector design for recombinant production of Olv2. The diagram depicts the gene of interest, inserted into the multicloning site (MCS) downstream of the T7 promoter, the ribosomal binding site, thioredoxin (TrxA) fusion partner, the Olv coding sequence, and a His-tag. The pET28a (+) plasmid carries a kanamycin resistance marker for selective propagation.

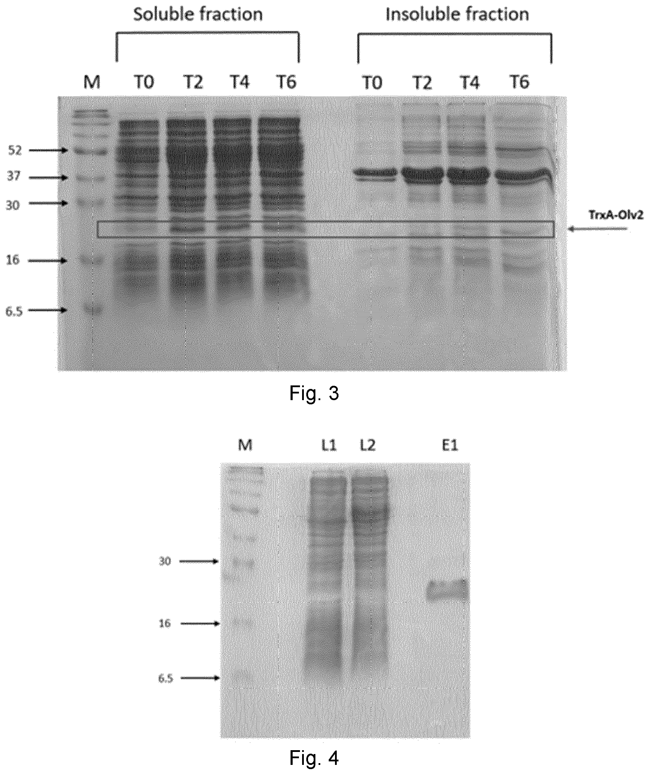

**Figure 3** shows recombinant expression of transformed *E.coli* (DE3), 30°C, 0.5mM IPTG over 6 hours (T₀ - T₆) soluble and insoluble fraction shown, the expected size of the fusion protein is 20 kDa. (M BlueEasy Prestained Protein Marker: T₀ - T₆ represent the time course samples taken both soluble and insoluble proteins shown.
**Figure 4** shows Ni NTA purification of cell lysate (soluble fraction) of transformed *E.coli* BI21 C41 (DE3), 30°C 0.5 mM IPTG 6 hours: Lane M: BlueEasy Prestained Protein Marker, Lane L1 is lysate before Ni-NTA, L2 is lysate post Ni-NTA purification, E1 is the direct elution from Ni-NTA.

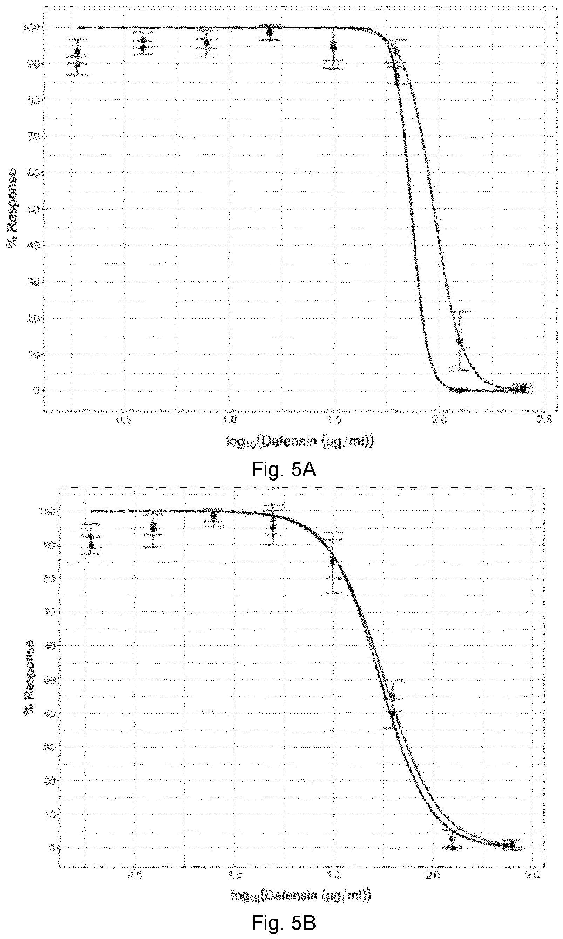

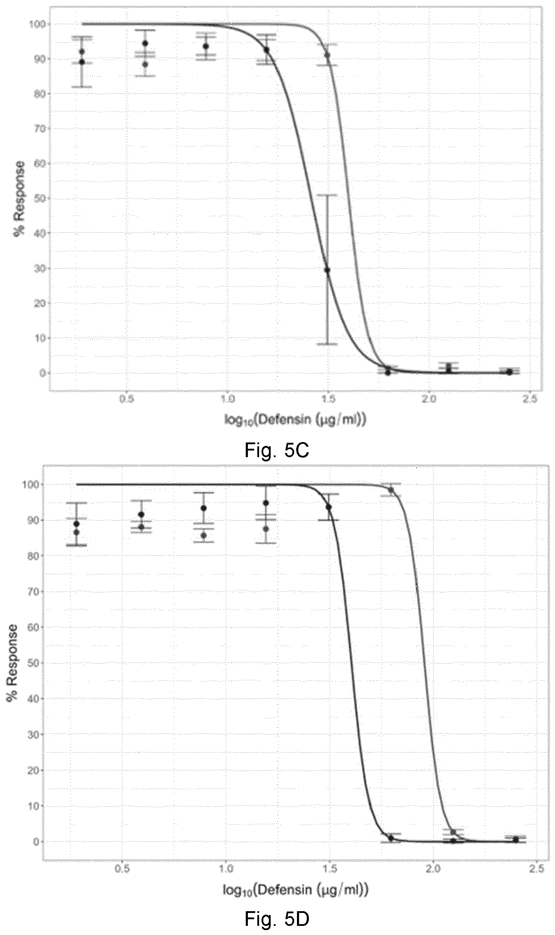

Figures 5A - D show graphs depicting the activity of Olv1 (right curve in the graphs) and Olv2 (left curve in the graphs) against *Candida albicans 5314* (fig. 5A), *Candida parapsilosis* (fig. 5B), *Candida tropicalis* (fig. 5C) and *Candida krusei* (fig. 5D). Dose-response curves were derived from three technical replicates (n = 3) and three biological replicates (N = 3) for each microbe.

### EXAMPLES

### Transcriptional identification of novel defensins

The extraction of RNA and generation of a cDNA library was conducted on seeds from *Olea europaea.* The 260/280 absorbance ratio for total RNA was 2.0 ± 0.05 for *Olea europaea* indicating pure RNA. The RNA was utilised to create a cDNA library, which was subsequently probed with primers illustrated in table 1. Primers were designed for a number of hits obtained using either a single method (e.g. Blast), a consensus of two methods (e.g. Levenshtein distance and HMMER 3) or finally, a consensus of all three methods. Table 1 next outlines the primers that were designed to amplify the sequences of the identified proteins, wherein the forward and reverse primers directed to 18S RNA are depicted as SEQ ID Nos 7 and 8, respectively, the forward and reverse primers directed to Olv1 SEQ ID Nos 9 and 10, respectively, and the forward and reverse primers directed to Olv2 SEQ ID Nos 11 and 12, respectively.

**Table 1: Primers designed for defensin-like proteins of O. europaea**

| PCR | | | |
|---|---|---|---|
| Target | **Primers sequence (5*** -> **3')** | **Expected Size** | **Size** |
| Ribosomal 18s | Forward 5'-ATTCTAGAGCTAATACGTGCAACAAACCCCGA-3' | **350** | **350** |
| RNA control | Reverse 5'-AGGATTGGGTAATTTGCGCGCC-3' | | |
| *O. europaea* | Forward 5'- TATGCAGCTGTGTTCTTCATGCTCATGC-3' | **245** | **250** |
| **Oly 1** | Reverse 5'- ACAATGTTTGGAGCAGAAGCAGCGG -3' | | |
| *O. europaea* | Forward 5'- TATCCTAGCTTATTTGCAACTGCTATGCTCGTG -3' | **260** | **270** |
| **Oly 2** | Reverse 5'- ACAGTGTTTGGTGCAGAAACAGCG -3' | | |

In order to check for the absence of false positives arising from genomic DNA contamination, cDNA synthesis was performed, without the reverse transcription enzyme, serving as a negative control to confirm any amplification originated from converted mRNA rather than gDNA contamination. As positive control for the PCR assay, ribosomal 18S RNA was amplified.

Figure 1 shows a robust amplification of the ribosomal 18S subunit in lane 1. In contrast; lane 2 reflects the negative control and exhibited no amplification, thereby confirming that the downstream amplification was attributable to cDNA rather than genomic DNA contamination. Lane 3 displayed modest amplification for a *O*. *europaea* protein Olv1 having the amino acid sequence SEQ ID No. 1. Notably, a more intense amplification was observed for another *O*. *europaea* protein Olv2 in lane 4, having the amino acid sequence SEQ ID No. 2.

### Cloning

The cloning process for both the Olv1 and Olv2 proteins were executed using advanced computational methods to optimize the gene's codons for efficient expression in an *Escherichia coli* host system (https://www.twistbioscience.com/). The original Olv1 gene sequence, designated as SEQ ID No. 3, was subject to this codon optimization, resulting in the modified sequence presented as SEQ ID No. 4. Both SEQ ID Nos. 3 and 4 are configured to encode the Olv1 protein, as outlined in SEQ ID No. 1. Similarly, the original Olv2 gene sequence, designated as SEQ ID No. 5, resulted in the modified optimal sequence presented as SEQ ID No. 6. Both SEQ ID Nos. 5 and 6 are configured to encode the Olv2 protein, as outlined in SEQ ID No. 2. The optimization processes were done to enhance the expression and stability of the Olv1 and Olv2 proteins in the *E*. *coli* system, thus facilitating the potential therapeutic action thereof.

The gene encoding Olv2 was cloned into a plasmid vector pET-28a(+) (Shilling et al. Communications Biology 3:2140 2020). The plasmid was constructed to provide a thioredoxin (TrxA) as a fusion protein partner, where the stop codon of thioredoxin was removed and a enterokinase cut site was added followed by the olv2 gene, followed by the plasmid His-tag for purification. The constructed plasmid is shown in figure 2. A similar cloning strategy was followed for constructing a corresponding pET-28a(+) based plasmid comprising the Olv1 coding sequence.

### Production of competent BL21 C41 (DE3) E.coli

28 g of nutrient agar powder was dissolved in 1l of deionised water and autoclaved at 121°C for 15 minutes, allow the solution to cool to 50°C and pour into sterile petri dishes. 12.5 g of LB-broth was dissolved in 500 ml of deionised water and autoclaved at 121°C for 15 minutes. 1.1 g of calcium chloride (anhydrous) was added to 80 ml of ultra-pure water. Once the calcium chloride solution dissolved, the solution was brought up to 100 ml with ultra-pure water to make a 100 mM CaCl₂ solution, stored at 4°C. 15 ml of 100% glycerol was added to 70 ml of ultra-pure water, 1.1 g calcium chloride (anhydrous) was added to the water-glycerol solution, and once dissolved, the solution was brought up to 100 ml to make a 100 mM CaCl₂ solution with 15% glycerol stored at 4°C.

A vial of C41 (DE3) *E.coli* cells was retrieved from -80°C storage and cultured on nutrient agar overnight at 37°C. A single colony from this plate was used to inoculate 15 ml of LB broth, which was then incubated at 37°C overnight in a shaking incubator. From this overnight culture, 200 µl was transferred to 50 ml of fresh LB media in a sterile conical flask and incubated at 37°C for 2 - 4 hours in a shaking incubator. The growth was monitored using a spectrometer 600 nm, aiming for an optical density of 0.5 - 0.6, indicative of log phase growth. The *E.coli* culture was then transferred to a 50 ml centrifuge tube, and spun at 5,000 g for 10 minutes. The supernatant was discarded and the cell pellet was resuspended in 15 ml of ice-cold 100 mM CaCl₂ solution and incubated overnight at 4°C. After a second centrifugation step at 5,000g for 10 minutes, the supernatant was again discarded, and the cells were resuspended in 15 ml of 100mM CaCl₂ with 15% glycerol. The calcium glycerol cell solution was aliquoted into 1.5 ml microfuge tubes - 500 µl each, and stored at -80°C. These cells are were now competent C41 (DE3) E.coli, ready for bacterial transformation.

### Plasmid transformation

28 g of nutrient agar powder was dissolved in 1l of deionised water and autoclaved at 121°C for 15 minutes, allowing the solution to cool to 50°C. The antibiotic was added aseptically to broth 100 µg/ml for ampicillin and 50 µg/ml for kanamycin, mixed and poured into sterile petri dishes.

Tryptone (1g, 2%), Yeast extract (250 mg, 0.5%), NaCl [30 mg, 10 mM], KCI [9.3 mg, 2.5 mM], MgCl [47.5mg, 10 mM], MgSO₄.7H2O [123.2 mg, 10mM] and Glucose [180 mg, 20mM] were added to a 100ml Duran glassware bottle. Ultra-pure water was added, and the pH was adjusted to 7.0 using 1M NaOH where necessary to a total volume of 50 ml and then autoclaved for 15 minutes at 121°C.

The plasmid yield was 1372 ng. To this, 686 µl of ultra-pure water was added, resulting in 2 ng/µl of plasmid DNA.

A vial of chemically competent C41 (DE3) *E.coli* cells were retrieved from the - 80°C storage and thawed on ice. Once thawed, cells were gently mixed and 50 µl aliquots were aseptically transferred into two sterile 1.5ml microcentrifuge tubes, kept on ice. To one tube, 5 µl of prepared plasmid DNA (10 ng) was added, while the second tube received 5 µl of ultra-pure water, serving as our control. The cells were carefully flicked 5 times to mix gently, then returned to ice for 30 minutes. This was followed by a heat shock treatment for 20 seconds in a pre-heated 42°C water bath, after which the tubes were placed back on ice for 5 minutes. Each tube was then diluted with 950 µl of room temperature SOC broth. The cells were incubated at 37°C for 1 hour in a shaking incubator (200rpm). Subsequently, 20 µl from each tube was streaked onto two nutrient agar plates, one containing 50 µg/ml kanamycin and the other without, and incubated overnight at 37°C. Additionally, 200 µl from each tube was inoculated into 5 ml of 2YT broth with 50 µg/ml kanamycin, serving as a transformation control.

BL21 C41 (DE3) expression stock: The following day, an isolated colony from the kanamycin-treated streak plate was inoculated into 5 ml sterile 2YT media containing 50 µg/ml kanamycin. The culture was grown for 3 - 5 hours until it reached an optical density (OD₆₀₀) of 0.7 - 0.8. Next, 500 µl aliquots of this culture were mixed with an equal volume of 50% glycerol solution, and frozen at -80°C in 1ml stock solutions for use in later expression.

### Recombinant Protein Expression

5 g IPTG was weighed and added to a 15 ml tube, and 8 ml of ultrapure water was added once dissolved. Additional ultrapure water was added to the tube for a total of 10 ml IPTG stock solution (500 mg/ml: 2 Molar) solution was sterile-filtered 0.2 µm.

KCI [466 mg, 25 mM] and Tris Base [378.5 mg, 12.5 mM] were dissolved in 240 ml ultrapure water in a 250ml Duran glassware bottle. The pH was adjusted to 7.5 using a 6M HCl solution, then filled to 250 ml followed by autoclaving 15 minutes 121°C.

Induction was eventually performed over several conditions, with different plasmids constructs and expression conditions. Optimised method: a BL21 C41 (DE3) stock solution containing the TrxA-OIv2 pET28a (+) plasmid (prepared as described in the plasmid transformation section) was thawed on ice for 1 hour. Next, 20 µl of this stock was aseptically transferred to 5 ml of 2YT media with 50 µg/ml kanamycin and incubated overnight at 37°C in a shaking incubator (180 rpm). The next day, 2 ml of the overnight culture was then mixed with 200 ml of 2YT media (50 µg/ml kanamycin) in a sterile 1l baffled conical flask (prepared by acid-washing, rinsing with deionised water, capping with cotton wool and tin foil, and autoclaving for 15 mins at 121°C. The flask was incubated at 37°C, 180 rpm for 3 - 4 hours until an OD₆₀₀ of 0.6 - 0.8 was reached. The temperature was subsequently lowered to 30°C. 50 µl of 500 mg/ml (2M) IPTG stock was aseptically added to the solution to achieve a final concentration of 0.5 mM IPTG. The flask was returned to the incubator at 30°C, 180 rpm for 6 hours. Every 2 hours, 1 ml aliquots were collected, transferred to 15 ml tubes and immediately centrifuged at 5,000 g for 10 mins. The supernatant was decanted, and the cell pellets were frozen at -20°C for subsequent lysis and analysis. After 6 hours, the remaining culture (200 ml) was divided into four 50 ml spin-down tubes, centrifuged at 5,000 g for 10 mins, resuspended in 10 ml of Bacterial Pellet Wash solution, and centrifuged again.. The clear supernatant was discarded, and the pelleted bacteria (x 4 tubes) were stored at -20°C for later protein extraction. The recombinant expression is shown in figure 3.

### Protein extraction, Ni-NTA purification & SDS-PAGE electrophoresis

Tris Base [1.51 g, 25 mM] and NaCl [7.305 g, 250 mM] were added to a 500 ml Duran glassware bottle and then dissolved in 475ml ultrapure water. The pH was adjusted to 8.3 using a 6 M HCl solution then filled to the 500 ml mark. The solution was stored at 4°C.

Ni NTA Buffer (9 ml) and glycerol (1 ml) were freshly mixed in a chilled 50 ml falcon tube followed by the addition of Triton-X-100 (100 µl) and PMSF (100 mM stock, 100 µl). The solution was chilled on ice for 40 minutes prior to use.

Nickel NTA resin (250 µl) was centrifuged (1000 g, 1min, RT) followed by removal of the storage buffer supernatant by careful pipetting. The supernatant was replaced by Ni NTA Buffer (125 µl) and mixed with the NTA beads. Isolation and replacement of the buffer was repeated three more times to equilibrate the Ni NTA beads.

27.2 µl of 500mg/ml imidazole stock solution was added to 9.5 ml Ni NTA Buffer. The solution was adjusted to pH 8.3 using a 3 M HCl solution, then filled to 10 ml using an additional Binding Buffer [20 mM imidazole].

680 µl of 500 mg/ml Imidazole stock solution [500 mM] was added to 9 ml Ni NTA Buffer. The solution was adjusted to pH 8.3 using a 3 M HCl solution, then filled to 10ml using an additional Binding Buffer.

SDS-PAGE electrophoresis was performed using a 15% SDS-Polyacrylamide separating gel. The gel, with a total volume 10 ml, was prepared using 2.3 ml of H₂O, 5 ml of 30% acrylamide, 2.5 ml of 1.5M Tris (pH 8.8), 100 µl of 10% sodium dodecyl sulphate (SDS) , and 100 µl ammonium persulfate (APS). In addition, a 4 ml 6% stacking gel was prepared using 2.65 ml H20, 0.8 ml of 30% acrylamide , 0.5 ml of 1 M tris (pH 6.8) 50 µl of 10% SDS , 40 µl of 10% APS and 4 µl TEMED. The gel was subjected to electrophoresis using a Bio-Rad Mini-PROTEAN^{®} Tetra Cell electrophoresis system, applying a constant voltage of 120 V for 90 mins.

Each of the frozen 1 ml time course cell pellet aliquots (15 ml tubes) was taken from the -20°C freezer and slowly thawed on ice for 1 hour, followed by the addition of lysis buffer (2 ml) and mixing by light mechanical stirring and aspiration. The bacteria were kept on ice for 40 mins, followed by sonication for 5 × 30 s (5 × 150 W) with 1-minute break intervals (on ice). The thick lysate solution was placed into two pre-chilled 2 ml microcentrifuge tubes for each time point, followed by centrifugation at 15,000 g for 15 mins 4°C. The clear yellow lysate supernatant was then pooled into 5ml tubes for each time point (0, 2, 4, 6 hours) and kept on ice. These represented our soluble fraction of proteins. The remaining pellets were subjected to 250 µl of 8 M Urea 25 mM DTT. The solution was vortexed and placed in a water bath for 10 mins at 80°C, this solution was then centrifuged at 10,000 g for 10 mins, and the supernatant was removed to a new 1.5 ml Eppendorf tube and represented an insoluble fraction. Both soluble and insoluble fractions of proteins were obtained for each time point 0 - 6 hours, ready for SDS sample prep and analysis.

The total cell pellet at the 6 hour end point was treated the same as each tested in the time course. Briefly: The remaining 200 ml that was split evenly into four 50 ml centrifuge tubes. These were removed from the -20°C freezer and slowly thawed on ice for 1 hour, followed by the addition of lysis buffer (10 ml) and mixing by light mechanical stirring and aspiration. The bacteria were kept on ice for 40 mins, followed by sonication for 5 × 30 s (5 × 150 W) with 1-minute break intervals (on ice). The thick lysate solution was placed evenly into pre-chilled 2ml microcentrifuge tubes followed by centrifugation at 15,000 g for 15mins 4°C. The supernatant was pooled into a pre-chilled 15 ml falcon. The resulting protein-rich lysate was equivalent to 6 - 8 ml. 250 µl of equilibrated Ni NTA beads were added to the lysate along with fresh PMSF protease inhibitor (100 mM stock, 100 µl). The falcon tube was then shaken for 2 hours (100 rpm, 4°C). The Ni NTA beads were then pelleted by gentle centrifugation (100 g, 1 min) followed by careful removal of the unbound lysate supernatant. The Ni NTA beads were resuspended in Wash Buffer and shaken for 1 hour. A second washing step was repeated. The washed Ni NTA beads were suspended in 1ml of Elution Buffer then transferred into a 2 ml microcentrifuge tube then shaken for 1 hour (130 rpm). The supernatant which contains the eluted protein was isolated after centrifugation (100 g, 1 min). Protein profile was identified by SDS-PAGE electrophoresis, shown in figure 4.

### Antimicrobial Testing

The proteins were chemically synthesised by solid phase peptide synthesis at a purity of > 90% by (GL Biochem Ltd. Shanghai, People's Republic of China). Post-synthesis, the proteins were stored lyophilised at -20°C to maintain their integrity. The proteins were weighed and dissolved in ultrapure water as a stock solution prior to experimentation. For all assays a 1 : 1 dilution was made with double-strength broth, the composition of which was tailored to the specific requirements of the organism.

Antifungal activity was assessed by a micro-broth dilution method modified by Handcock for cationic peptides and proteins. Given the propensity of cationic peptides and proteins to bind polystyrene, 96-well polypropylene plates were used as an alternative. *Candida* species MIC analysis were performed using ½ strength potato dextrose broth (PDB).

96-well flat-bottomed polypropylene plates were prepared 30 mins prior to inoculation. The outer wells of the plate were filled with 200 µl of diH₂O to mitigate evaporation in test lanes during incubation with low volumes and for prolonged periods. Given the high cost of the synthetic proteins minimal volume of 100 µl was utilised, and preliminary testing was conducted in duplicate. 50 µl of ½ strength PDB was dispensed into all remaining wells except column 3. 100 µl was added to column 2 and acting as a 'broth only' sterile control. Subsequently, 100 µl of the top standard protein solution, prepared at a 2x concentration (500 µg/ml) and suspended in half-strength PDB, was added to column 3. Using a multichannel pipette, 50 µl from each well in column 3 (rows B through G) were mixed with an equal volume of broth in column 4. This procedure was repeated by serial diluting the protein down to column 10. The final 50µl was not transferred to lane 11 but was instead disposed of.

*Candida* species were grown over 48 hours on ½ strength potato dextrose agar (PDA) at a temperature of 35°C. Cells were harvested directly from the agar plates and resuspended in 2 ml of ½ PDB. The cell concentration was determined using a haemocytometer, and subsequently, cells were diluted to 1 × 10⁵ cells/ml in ½ strength PDB, resulting in a concentration of 5,000 cells per well. 50 µl of the diluted cell suspension was added to the wells in columns 3 - 11, working backwards from the lowest protein concentration. This step ensured a total volume of 100 µl in each well, giving a final top standard protein concentration of 250 µg/ml in the assay. Column 11 was designated as the growth control, containing cells only.

Absorbance readings were taken at time zero (T=0) at a wavelength of 595 nm for the analysis of all *Candida* spp. Subsequently, all plates were incubated at 35°C for 24 hrs. To ensure uniform resuspension of cells, a multichannel pipette was used to agitate the cell suspension. A second series of absorbance measurements at 595 nm was conducted at T = 24, enabling a comprehensive assessment of growth over the 24-hour period).

The activities of synthetic plant-like defensin analogues Olv1 and Olv2 were assessed against *Candida albicans* sc5314 (Table 2, Figure 5A), *Candida parapsilosis* (Table 3, Figure 5B), *Candida tropicalis* (Table 4, Figure 5C) and *Candida krusei* (Table 5, Figure 5D).

LogICso: The logarithm of the concentration at which 50% inhibition of growth is observed, Hill-slope: Describes the steepness of the curve; a steeper slope indicates a more cooperative binding to the target and a sharper transition between the non-effective and effective doses and IC₅₀: The actual concentration at which 50% inhibition is achieved, derived from the LogIC₅₀.

### Candida albicans

For Olv1, the loglC50 is 1.974, meaning the IC50, or the concentration at which the response is half of the maximum, is 94.113 µg/ml. The Hill-slope of -6.442 suggests that the response decreases steeply as the concentration of Olv1 increases. A steep Hill-slope (absolute value significantly greater than 1) can indicate positive cooperativity in binding, meaning multiple molecules of Olv1 might bind to a target site to exert its effect. The tight confidence intervals and the highly significant p-values for the logIC₅₀ (p < 0.001) and Hill-slope (p < 0.05) indicate a high level of precision and reliability in these estimates.

**Table 2: Dose-response parameters and statistical analysis for plant defensin-like proteins derived from Olea europaea: Olv1 and Olv2 against Candida albicans 5314.**

| | **Olv1** | | | **Olv2** | | |
|---|---|---|---|---|---|---|
| **Parameter** | **LogIC₅₀** | **Hill-slope** | **IC₅₀** | **LogIC₅₀** | **Hill-slope** | **IC₅₀** |
| **Estimate** | 1.974 | -6.443 | 94.113 | 1.866 | -11.59 | 73.46 |
| **Std. Error** | 0.029 | 1.353 | 6.23 | 0.193 | 31.772 | 32.59 |
| **t-value** | 68.65 | -4.763 | NA | 9.685 | -0.365 | NA |
| **p-value** | <0.001 | 0.003 | NA | <0.001 | 0.728 | NA |
| **95% Cl (Lower)** | 1.899 | NA | NA | NA | NA | NA |
| **95% Cl (Upper)** | 2.13 | -4.151 | NA | 1.981 | -9.801 | NA |

For Olv2, the logIC₅₀ is slightly lower at 1.866, indicating a slightly more potent effect with an IC50 of 73.460 µg/ml. However, the Hill-slope for Olv2 is -11.590, which is steeper than that of Olv1. This suggests even stronger positive cooperativity or a more pronounced effect per molecule when binding. It should be noted, however, that the standard error and confidence interval for Olv2's Hill-slope are much larger, and the p-value is not significant (p > 0.05), indicating less reliability in this estimate. This could mean that the data for Olv2 is more variable or that there are more outliers affecting the slope estimate.

The dose-response curve visually supports these interpretations (Figure 1), with the steepness of each curve corresponding to the Hill-slope values and the shift to the left for Olv2 indicating its higher potency.

### Candida parapsilosis

For Olv1, the LogICso value is 1.755, which translates to an IC₅₀ value of 56.919 µg/ml, indicating the concentration at which the compound inhibits 50% of the biological activity. A Hill-slope of -3.181 suggests that the response is reasonably cooperative, but less so than what was observed with the previous dataset for Candida albicans. The steepness is moderate, indicating a standard sigmoidal dose-response curve. The standard error is low, indicating precision in these estimates, and the highly significant p-value (<0.001) suggests that the estimates are statistically robust. The 95% confidence interval for the LogIC₅₀ is narrow (from 1.700 to 1.807), which reinforces the precision.

For Olv2, the LogIC₅₀ value is 1.734 with an IC₅₀ of 54.233 µg/ml, making it slightly more potent than Olv1 in this data set. The Hill-slope is -3.474, which is very slightly steeper than that of Olv1, suggesting a more cooperative binding in this context compared to Olv1. This could imply that Olv2 has a slightly different mechanism or a higher affinity for its target. The standard error and the 95% confidence intervals for Olv2 are also low and narrow, indicating good precision and reliability. The highly significant p-values (<0.001) for both the LogICso and Hill-slope indicate that these results are statistically significant.

**Table 4: Dose-response parameters and statistical analysis for plant defensin-like proteins derived from Olea europaea: Olv1 and Olv2 against Candida parapsilosis.**

| | **Olv1** | | | **Olv2** | | |
|---|---|---|---|---|---|---|
| **Parameter** | **LogIC₅₀** | **Hill-slope** | **IC₅₀** | **LogIC₅₀** | **Hill-slope** | **IC₅₀** |
| **Estimate** | 1.755 | -3.181 | 56.919 | 1.734 | -3.474 | 54.233 |
| **Std. Error** | 0.021 | 0.465 | 2.786 | 0.026 | 0.659 | 3.227 |
| **t-value** | 82.565 | -4.763 | NA | 67.110 | -5.271 | NA |
| **p-value** | <0.001 | <0.001 | NA | <0.001 | <0.001 | NA |
| **95% Cl (Lower)** | 1.700 | -5.078 | NA | 1.667 | -11.872 | NA |
| **95% Cl (Upper)** | 1.807 | -2.309 | NA | 1.796 | -2.304 | NA |

The associated dose-response curve shows two overlapping sigmoidal curves with the Olv2 curve being slightly to the left of Olv1, representing its higher potency. Both curves would exhibit a standard sigmoidal shape given their Hill-slopes closer to - 1, which indicates a less complex interaction with their target than the previous dataset suggested.

### Candida tropicalis

For the defensin-like protein Olv1, derived from Olea europaea, the reported LogICso value stands at 1.602, corresponding to an IC50 value of approximately 39.975 µg/ml. The Hill-slope of -9.410 for Olv1 is indicative of a highly cooperative interaction, albeit the statistical significance of this cooperativity is not substantiated (p-value = 0.236), suggesting that the steepness observed may not be as reliable as the LogIC₅₀ value, for which the statistical robustness is confirmed by a p-value of <0.001.

In contrast, the Olv2 protein exhibits a LogIC₅₀ value of 1.417, which equates to an IC50 of 26.098 µg/ml. This denotes a slightly higher potency relative to Olv1. The Hill-slope of-4.862 for Olv2, though less steep than that of Olv1, is statistically significant (p-value = 0.006), implying a reliable estimate of cooperativity in its binding mechanism. The observed cooperative nature of Olv2 may suggest a distinct interaction dynamic or a heightened affinity for the biological target, compared to Olv1.

**Table 5: Dose-response parameters and statistical analysis for plant defensin-like proteins derived from Olea europaea: Olv1 and Olv2 against Candida tropicalis.**

| | **Olv1** | | | **Olv2** | | |
|---|---|---|---|---|---|---|
| **Parameter** | **LogIC₅₀ (µg/ml)** | **Hill-slope** | **IC₅₀ (µg/ml)** | **logIC₅₀ (µg/ml)** | **Hill-slope** | **IC₅₀ (µg/ml)** |
| **Estimate** | 1.602 | -9.410 | 39.975 | 1.417 | -4.862 | 26.098 |
| **Std. Error** | 0.084 | 7.147 | 7.734 | 0.026 | 1.183 | 1.546 |
| **t-value** | 19.065 | -1.317 | NA | 55.055 | -4.109 | NA |
| **p-value** | <0.001 | 0.236 | NA | <0.001 | 0.006 | NA |
| **95% Cl (Lower)** | NA | NA | NA | 1.351 | NA | NA |
| **95% Cl (Upper)** | NA | -4.467 | NA | NA | -2.929 | NA |

### Candida krusei

Olv1 exhibits a LogIC₅₀ value of 1.957, which is converted to an IC₅₀ value of 90.642 µg/ml. This implies that a higher concentration of Olv1 is required to inhibit 50% of the biological activity compared to its efficacy against Candida tropicalis, suggesting reduced potency against *C*. *krusei.* The Hill-slope of -11.209, while indicative of a very steep dose-response relationship, does not confer statistical significance in this context (p-value < 0.001).

Olv2 however possesses higher potency corresponding to an IC₅₀ of 40.346 µg/ml, relative to Olv1 against *C*. *krusei.* However, the Hill-slope of -10.527 for Olv2, although steep and suggestive of cooperative binding, lacks statistical significance (p-value = 0.329).

The t-values, albeit lower than those for the previous dataset, still imply a significant deviation from the null hypothesis for the LoglCso estimates, corroborated by p-values of <0.001 for both proteins.

**Table 5: Dose-response parameters and statistical analysis for plant defensin-like proteins derived from Olea europaea: Olv1 and Olv2 against Candida krusei.**

| | **Olv1** | | | **Olv2** | | |
|---|---|---|---|---|---|---|
| **Parameter** | **LogIC₅₀ (µg/ml)** | **Hill-slope** | **IC₅₀ (µg/ml)** | **LogIC₅₀ (µg/ml)** | **Hill-slope** | **IC₅₀ (µg/ml)** |
| **Estimate** | 1.957 | 11.209 | 90.642 | 1.606 | 10.527 | 40.346 |
| **Std. Error** | 0.158 | 12.189 | 33.005 | 0.107 | 9.915 | 9.951 |
| **t-value** | 12.377 | -0.920 | NA | 14.991 | -1.062 | NA |
| **p-value** | <0.001 | <0.001 | NA | <0.001 | 0.329 | NA |
| **95% Cl (Lower)** | NA | NA | NA | NA | NA | NA |
| **95% Cl (Upper)** | NA | -3.449 | NA | NA | -5.595 | NA |

Overall, it is clear that both OIv1 and Olv2 exhibit potent antifungal activities against a number of *Candida* spp..

Plant defensins have previously been shown to be sensitive to salt, which can be attributed to a few key reasons:

Structure-Function Relationship: Plant defensins have a specific three-dimensional structure stabilized by disulfide bridges, essential for their interaction with fungal membranes or cell wall components. High salt concentrations can disrupt these structures, leading to a loss of function. The ionic environment influences the conformational stability of these proteins, impacting their ability to bind to and permeate fungal cell walls.

Interaction with Membrane Components: The antifungal mechanism of many defensins often involves interacting with specific membrane lipids in fungi. Salts can interfere with these interactions either by shielding the charge on the defensins or the fungal membrane components, or by altering the membrane fluidity and composition, thus hindering the defensin's ability to exert its antifungal effect.

### Other microbes

In addition to the *Candida* spp. tested, the growth inhibitory effects of Olv1 and Olv2 against a panel of relevant pathogens was tested, see table 8.

**Table 8. Growth Inhibitory effects of Olive Seed Defensins against pathogen panel.**

| Organism | Olv1 (µg/ml) | Olv2 (µg/ml) |
|---|---|---|
| *E.coli 0157* | ND | 73 |
| *B. cereus* | 43 | 19 |
| *T. rubrum* | ND | 228 |
| *T. interdigitale* | ND | 24 |
| *P. aeruginosa* | ND | 289 |

| | | |
|---|---|---|
| ND: Not determined | | |

Table 8 depicts the IC50 values (ug/ml) calculated following testing of Olv1 and Olv2 against additional relevant disease causing pathogens.

In the context of antimicrobial research, the following pathogens are assessed, due to their significant implications for human health and their notable resistance to conventional treatments:

*Escherichia coli* O157: This particular strain of *Escherichia coli* is recognized for its role in serious foodborne illnesses. It is characterized by the production of Shiga toxins, leading to severe gastrointestinal disturbances and potentially life-threatening complications such as haemolytic uremic syndrome (HUS). The pathogen's association with a variety of food products, including undercooked meats and unprocessed produce, underscores its public health relevance.

*Bacillus cereus:* As an agent of foodborne illness, *Bacillus cereus* is known for producing toxins that cause distinct gastrointestinal symptoms, categorized into diarrheal and emetic syndromes. The pathogen's prevalence in improperly stored or cooked foods, including various starch-rich foods, necessitates consideration in food safety protocols.

*Trichophyton rubrum:* This dermatophyte fungus is primarily implicated in cutaneous infections such as tinea pedis (athlete's foot) and onychomycosis. Its contagious nature, particularly in moist communal environments, presents a significant challenge in dermatological and public health contexts.

*Trichophyton interdigitale*: Similar to *Trichophyton rubrum, Trichophyton interdigitale* contributes to dermatophytic infections, including athlete's foot and ringworm. The fungus thrives in damp environments, leading to its widespread prevalence and the necessity for effective antifungal interventions.

*Pseudomonas aeruginosa:* Notable for its role in nosocomial infections, *Pseudomonas aeruginosa* is a pathogen of major concern, especially among immunocompromised patients. It is capable of causing diverse infections, ranging from pneumonia to wound and urinary tract infections. The bacterium's notable antibiotic resistance complicates treatment strategies, making it a critical focus in antimicrobial research.

## Claims

1. Antimicrobial protein, chosen from the group, consisting of:
a. a protein comprising the amino acid sequence of SEQ ID No 1,
b. a protein comprising the amino acid sequence of SEQ ID No 2, and
c. a protein, being a functional analogue of the protein of a. or b., comprising an amino acid sequence having at least 90% amino acid sequence homology with that of SEQ ID No 1 or SEQ ID No 2.

2. Antimicrobial protein of claim 1, being derived from *Olea europea.*

3. Nucleic acid sequence, encoding the protein according to claim 1 or 2.

4. Gene, encoding the protein of claim 1 or comprising the nucleic acid sequence of claim 2.

5. Nucleic acid vector, comprising the nucleic acid sequence of claim 2 or the gene of claim 3.

6. Host cell, comprising the nucleic acid sequence of claim 3 or the gene of claim 4, the said nucleic acid sequence or gene being exogenous to the host cell.

7. Host cell, being transformed with the nucleic acid vector of claim 5.

8. Host cell of claim 6 or 7, belonging to the genus *Escherichia.*

9. Composition, comprising the antimicrobial protein of any of claims 1-3, optionally wherein the composition further comprises a carrier such as a pharmaceutically acceptable carrier.

10. Use of the antimicrobial protein of claim 1 or 2, or the composition of claim 9 as an antiseptic agent.

11. Antimicrobial protein of claim 1 or 2 for use for treatment of a microbial infection in a subject, for example a patient, in need thereof.

12. Antimicrobial protein of claim 11 wherein the antimicrobial infection is an infection by one or more of *Candida, E.coli, B. cereus, T. rubrum, T. interdigitale* and *P*. *aeruginosa.*

13. A method of treating a subject for a microbial infection comprising the step of administering to the subject the antimicrobial protein of claim 1 or 2, or the composition of claim 9; optionally wherein the administration is by topical application.
